(19) Europäisches Patentamt
European Patent Office
Office européen des brevets

(11) EP 0 639 179 B1

(12) # EUROPÄISCHE PATENTSCHRIFT

(45) Veröffentlichungstag und Bekanntmachung des Hinweises auf die Patenterteilung:
**29.05.1996 Patentblatt 1996/22**

(21) Anmeldenummer: **92903336.3**

(22) Anmeldetag: **20.01.1992**

(51) Int Cl.⁶: **C07C 401/00**, A61K 31/59

(86) Internationale Anmeldenummer:
**PCT/EP92/00123**

(87) Internationale Veröffentlichungsnummer:
**WO 92/12963 (06.08.1992 Gazette 1992/21)**

(54) **23-OXA-DERIVATE IN DER VITAMIN-D-REIHE, VERFAHREN ZU IHRER HERSTELLUNG, DIESE DERIVATE ENTHALTENDE PHARMAZEUTISCHE PRÄPARATE SOWIE DEREN VERWENDUNG ALS ARZNEIMITTEL**

23-OXA DERIVATIVES OF D-SERIES VITAMINS, METHOD OF PREPARING THEM, PHARMACEUTICAL PREPARATIONS CONTAINING THEM, AND THE USE OF SUCH PREPARATIONS AS DRUGS

DERIVES 23-OXA DE VITAMINES DE LA SERIE D, PROCEDE DE PREPARATION DESDITS DERIVES, PREPARATION PHARMACEUTIQUE CONTENANT CES DERIVES, ET UTILISATION DESDITES PREPARATIONS COMME MEDICAMENTS

(84) Benannte Vertragsstaaten:
**AT BE CH DE DK ES FR GB GR IT LI LU MC NL SE**

(30) Priorität: **19.01.1991 DE 4101953**

(43) Veröffentlichungstag der Anmeldung:
**22.02.1995 Patentblatt 1995/08**

(73) Patentinhaber: **SCHERING AKTIENGESELLSCHAFT**
**D-13342 Berlin (DE)**

(72) Erfinder:
- **NEEF, Günter**
  **D-1000 Berlin 31 (DE)**
- **STEINMEYER, Andreas**
  **D-1000 Berlin 19 (DE)**
- **KIRSCH, Gerald**
  **D-1000 Berlin 33 (DE)**
- **SCHWARZ, Katica**
  **D-1000 Berlin 10 (DE)**
- **HABEREY, Martin**
  **D-1000 Berlin 46 (DE)**
- **THIEROFF-EKERDT, Ruth**
  **D-1000 Berlin 28 (DE)**
- **RACH, Petra**
  **D-1000 Berlin 65 (DE)**

(56) Entgegenhaltungen:
**EP-A- 0 078 704** **EP-A- 0 184 112**
**WO-A-90/09992** **WO-A-91/15475**

- **TETRAHEDRON LETTERS. Bd. 32, Nr. 38, 16. September 1991, OXFORD GB Seiten 5073 - 5076; G. NEEF ET AL: 'Synthesis of 23-Oxa Calcitriol Derivatives'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft 23-Oxa-Derivate in der Vitamin D-Reihe der Formel I

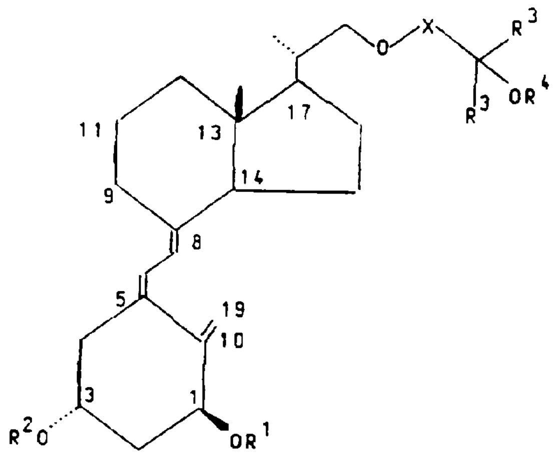

(I),

worin

$R^1$, $R^2$ und $R^4$ unabhängig voneinander ein Wasserstoffatom oder eine Acylgruppe mit 1 bis 9 Kohlenstoffatomen,
$R^3$ je ein Wasserstoffatom oder je eine lineare oder verzweigte Alkylgruppe mit 1 bis 4 Kohlenstoffatomen und X einen Alkylenrest-$(CH_2)_n$- mit n = 1, 2, 3, wobei wenn n = 1 $R^3$ nicht je eine Methyl- oder Propylgruppe, letztere in Verbindung mit $R^1$, $R^2$ und $R^4$ in der Bedeutung je einer $C_1$-$C_9$-Acylgruppe sein kann, bedeuten,
sowie ein Verfahren zu deren Herstellung, pharmazeutische Präparate, die diese Verbindungen enthalten sowie deren Verwendung zur Herstellung von Arzneimitteln.

Die für die Reste $R^1$, $R^2$ und $R^4$ möglichen Acylgruppen sind insbesondere von gesättigten oder auch von ungesättigten, geradkettigen oder verzweigten Carbonsäuren oder der Benzoesäure abgeleitet.

Als Alkylgruppen für $R^3$ kommen in erster Linie die Methyl-, Ethyl- oder Propylgruppe infrage.

Bevorzugt gemäß vorliegender Erfindung sind 23-Oxa-Vitamin-D-Derivate der allgemeinen Formel I, in welchen $R^1$, $R^2$ und $R^4$ für ein Wasserstoffatom und $R^3$ je für eine Ethylgruppe stehen.

Besonders bevorzugt sind die Verbindungen
24-(1-Ethyl-1-hydroxypropyl)-23-oxa-9,10-seco-5(Z),7(E),10(19)-cholatrien-1α,3β-diol.
24-(1-Hydroxy-1-propylbutyl)-23-oxa-9,10-seco-5(Z),7(E),10(19)-cholatrien-1α,3β-diol.
24-(3-Hydroxy-3-methylbutyl)-23-oxa-9,10-seco-5(Z),7(E),10(19)-cholatrien-1α,3β-diol.
24-(3-Ethyl-3-hydroxypentyl)-23-oxa-9,10-seco-5(Z),7(E),10(19)-cholatrien-1α,3β-diol.

Vitamin D-Derivate, die in 1- und 3-Position wie die erfindungsgemäßen Verbindungen substituiert und die Seitenkette

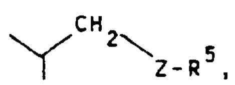

worin Z u.a. für ein

Sauerstoffatom und $R^5$ für eine gerad- oder verzweigtkettige, gegebenenfalls substituierte aliphatische Gruppe mit 1 bis 12 Kohlenstoffatomen steht, sein können/kann, sind in der EP-A-0078 704 beschrieben. Besonders bevorzugt sind dabei die Verbindungen, worin Z u.a. ein Sauerstoffatom und $R^5$ die Gruppe $-CH_2-C(CH_3)_2OR^6$ ($R^6$ = H, Hydroxyschutzgruppe) bedeutet.

Die natürlichen Vitamine $D_2$ und $D_3$ (vgl. allgemeine Formel VIII) sind an sich biologisch inaktiv und werden erst nach Hydroxylierung in 25-Position in der Leber bzw. in 1-Position in der Niere in deren biologisch aktive Metaboliten umgewandelt. Die Wirkung der Vitamine $D_2$ und $D_3$ besteht in der Stabilisierung des Plasma-$Ca^{++}$ - und Plasma-Phosphat-Spiegels; sie wirken einem Absinken des Plasma-$Ca^{++}$-Spiegel entgegen.

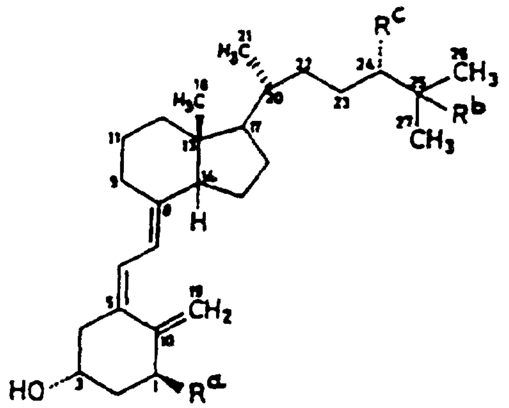

(VIII)

Ergocalciferol: $R^a=R^b=H, R^c=CH_3$, Vitamin $D_2$
Doppel bindung C-22/23
Cholecalciferol: $R^a=R^b=R^c=H$ Vitamin $D_3$
25-Hydroxycholecalciferol: $R^a=R^c=H, R^b=OH$
1α-Hydroxycholecalciferol: $R^a=OH, R^b=R^c=H$
1α,25-Dihydroxycholecalciferol: $R^a=R^b=OH, R^c=H$ Calcitriol

Neben ihrer ausgeprägten Wirkung auf den Calcium- und Phosphatstoffwechsel besitzen Vitamin $D_2$ und $D_3$ und seine synthetischen Abkömmlinge auch proliferationshemmende und zelldifferenzierende Wirkungen (H.F. De Luca, The Metabolism and Function of Vitamin D in Biochemistry of Steroid Hormones. Hrsg. H.L.J. Makin, 2nd Edition, Blackwell Scientific Publications 1984, S. 71-116). Bei Vitamin D-Anwendung kann es aber zu Überdosierungserscheinungen kommen (Hypercalcämie).

In 24-Stellung hydroxylierte 1α-Cholecalciferole gehen bereits aus der DE-AS-25 26 981 hervor; sie besitzen eine geringere Toxizität als das entsprechende nicht-hydroxylierte 1α-Cholecalciferol. Die hydroxylierten Verbindungen zeigen eine selektive Aktivierung der intestinalen Calciumabsorption und eine schwächere Knochenabsorptionswirkung als 1α-Cholecalciferol.

Die in der internationalen Patentanmeldung WO 87/00834 beschriebenen 24-Hydroxy-Vitamin-D-Analoga können für die behandlung von durch abnormer ZellProliferation und/oder Zelldifferentiation hervorgerufenen Störungen beim Menschen und Tier dienen.

Für verschiedene 1,25-Dihydroxy-Homo-Vitamin-D-Derivate ist eine Dissoziation bezüglich der Eigenschaften Knochenabsorptionswirkung und HL-60 Zelldifferentiation schon kürzlich von De Luca erwähnt worden. Die Knochenabsorptionswirkung in vitro ist dabei ein direktes Maß für die Calciummobilisierung in vivo.

Es wurde nun gefunden, daß die erfindungsgemäßen 23-Oxa-Vitamin-D-Derivate der allgemeinen Formel I im Vergleich zum Vitamin-D-Abkömmling Calcitriol (1α,25-Dihydroxycholecalciferol) überraschenderweise ein günstigeres Wirkungsspektrum aufweisen. Während die Effekte auf den Calcium- und Phosphatstoffwechsel deutlich abgeschwächt sind (Verringerung der Nebenwirkungen durch Überdosierung oder erforderlicher hoher Dosierung), bleiben die proliferationshemmenden und zelldifferenzierenden Wirkungen annähernd erhalten (Dissoziation).

Die Vitamin-D-Aktivität der erfindungsgemäßen Verbindungen wird mittels des Calcitriol-Rezeptortests bestimmt. Er wird unter Verwendung eines spezifiscnen Rezeptorproteins aus dem Darm von jungen Schweinen durchgeführt (siehe Dame, M.C.; Pierce, E.A.; DeLuca, H.F., Proc. Natl. Acad. Sci. USA, 82, 7825 (1985)). Rezeptorhaltiges Bindungsprotein wird mit einer ethanolischen Lösung von $^3H$-Calcitriol (0.025μCi) in einem Reaktionsvolumen von 0,25 ml in Abwesenheit und in Anwesenheit der Prüfsubstanzen für zwei Stunden in einem Teströhrchen inkubiert. Zur Trennung von freiem und rezeptorgebundenem Calcitriol wird eine Charcoal-Dextran-Absorption durchgeführt. Dazu werden 250 μl einer Charcoal-Dextran-Suspension jedem Teströhrchen zugeführt und bei 4°C für 20 Minuten inkubiert. Anschließend werden die Proben bei 1500 x g 10 Minuten bei 4°C zentrifugiert. Der Überstand wird dekantiert und nach ca. 1stündiger Äquilibrierung in Atom-Light in einem β-Zähler gemessen.

Die mit verschiedenen Konzentrationen der Prüfsubstanz sowie der Referenzsubstanz (unmarkiertes Calcitriol) bei konstanter Konzentration der Bezugssubstanz ($^3H$-Calcitriol) erhaltenen Kompetitionskurven werden in Beziehung zueinander gesetzt und ein Kompetitionsfaktor (KF) ermittelt.

Er ist definiert als Ouotient aus den Konzentrationen der jeweiligen Prüfsubstanz und der Referenzsubstanz, die für 50%ige Kompetition erforderlich sind:

$$KF = \frac{\text{Konzentration Prüfsubstanz bei 50\% Kompetition}}{\text{Konzentration Referenzsubstanz bei 50\% Kompetition}}$$

Demnach besitzen

24-(1-Ethyl-1-hydroxypropyl)-23-oxa-9,10-seco-5(Z), 7(E),10(19)-cholatrien-1α,3β-diol (6a)

24-(1-Hydroxy-1-propylbutyl)-23-oxa-9,10-seco-5(Z),7(E),10(19)-cholatrien-1α,3β-diol (6b) sowie
24-(3-Hydroxy-1-methylethyl)-23-oxa-9,10-seco-5(Z),7(E),10(19)-cholatrien-1α,3β-diol (6c)
KF-Werte von 2,2 (6a), 4,5 (6b) bzw. 4,6 (6c).
Diese Daten zeigen, daß die beschriebenen Verbindungen etwa gleich stark wie Calcitriol an dessen Rezeptor binden.
Die erfindungsgemäßen Verbindungen zeigen eine signifikante Orthokeratose-Induktion am Mäuseschwanz ohne Einfluß auf den $Ca^{2+}$-Stoffwechsel zu nehmen. Die Vergleichsverbindung 6c beeinflußt bei einer analogen Stimulierung des Stratum granulosum den Calcium-Stoffwechsel erheblich.
Der Mäuseschwanz weist physiologischerweise neben Bereichen mit orthokeratotischer Verhornung auch parakeratotisches Stratum corneum auf. Durch topische Applikation von Calcitriolanaloga und weiteren Substanzen, wie z. B. Vitamin A-Säure und dem antipsoriatisch wirksamen Dithranol, läßt sich eine orthokeratotische Verhornung der parakeratotischen Haut induzieren. Meßparameter ist die prozentuale Zunahme des Anteils von Epidermisregionen, die im histologischen Präparat ein Stratum granulosum als Marker der Orthokeratose haben.
(R. Wrench: Assessing Drugs for Psoriasisform Diseases and their Antiparakeratotic Mechanisms Using the Mouse Tail Test; Maibach, Lowe (eds.) Models in Dermatology, vol. 2, pp 76-91 (Karger, Basel 1985)) Gleichzeitig wird in diesem Modell durch Bestimmung der Calciumkonzentration im Serum die Dissoziation zwischen lokaler wachstumsregulierender und systemischer calcitroper Wirkung beobachtet (Ergebnisse siehe Tabelle 1).
Tierart: Maus; Geschlecht: weiblich; Applikationsart: topisch; Applikationsdauer: 19 Tage; Applikationsvolumen: 0.05 ml 1x täglich Montag-Freitag; Formulierung: gelöst in Ethanol/Isopropylmyristat; Dosierung der Prüfsubstanz: 0,005 %. Durch das verminderte Hypercalcämie-Risiko eignen sich die erfindungsgemäßen Substanzen in besonderer Weise zur Herstellung von Arzneimitteln für die Behandlung von Erkrankungen, die durch eine Hyperproliferation gekennzeicnnet sind, z. B. hyperproliferative Erkrankungen der Haut (Psoriasis) und maligne Tumoren (Leukämie, Coloncarcinom, Mammacarcinom).

In einer besonders bevorzugten Ausführungsform der Erfindung werden vor der Behandlung im Zielorgan Calcitriolrezeptoren nachgewiesen.

Die vorliegende Erfindung bezieht sich somit auch auf pharmazeutische Präparate, die mindestens eine Verbindung gemäß der allgemeinen Formel I zusammen mit einem pharmazeutisch verträglichen Träger enthalten. Die Verbindungen können formuliert werden als Lösungen in pharmazeutisch verträglichen Solventien oder als Emulsionen. Suspensionen oder Dispersionen in geeigneten pharmazeutischen Solventien oder Trägern oder als Pillen, Tabletten oder Kapseln, die in an sich bekannter Weise feste Trägerstoffe enthalten. Für eine topische Anwendung werden die Verbindungen vorteilhafterweise als Cremes oder Salben oder in einer ähnlichen, zur topischen Anwendung geeigneten Arzneimittelform formuliert. Jede derartige Formulierung kann auch andere pharmazeutisch verträgliche und nichttoxische Hilfsstoffe enthalten, wie z. B. Stabilisatoren, Antioxidantien, Bindemittel, Farbstoffe, Emulgatoren oder Geschmackskorrigentien. Die Verbindungen werden vorteilhafterweise durch Injektion oder intravenöse Infusion geeigneter steriler Lösungen oder als orale Dosierung über den Ernährungstrakt oder topisch in der Form von Cremes, Salben, Lotions oder geeigneter transdermaler Pflaster appliziert, wie in der EP-A-0387 077 beschrieben ist.

Die tägliche Dosis liegt bei

0,1 µg/Patient/Tag - 1000 µg (1 mg)/Patient/Tag,

vorzugsweise

1.0 µg/Patient/Tag - 500 µg/Patient/Tag.

Außerdem betrifft die Erfindung die Verwendung der Verbindungen gemäß Formel I zur Herstellung von Arzneimitteln.

Die Herstellung der Verbindungen der allgemeinen Formel I', (das sind die Verbindungen der allgemeinen Formel I sowie diejenigen Verbindungen, die durch die Weglassung des disclaimers in der allgemeinen Formel I hinzukommen) werden nach einem neuen Verfahren hergestellt.

Die Erfindung betrifft daher auch ein Verfahren zur Herstellung von Verbindungen der allgemeinen Formel I'

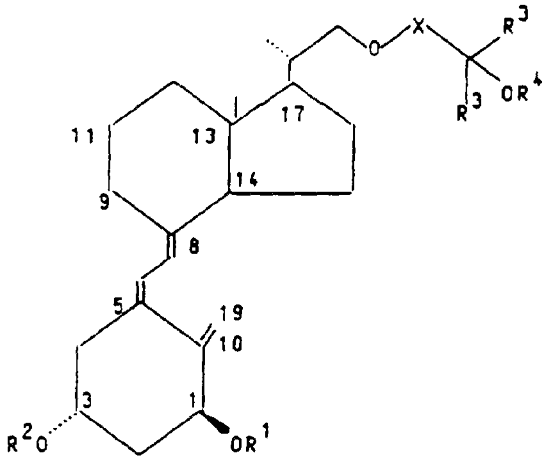

(I´)

worin

$R^1$, $R^2$ und $R^4$ unabhängig voneinander ein Wasserstoffatom oder eine Acylgruppe mit 1 bis 9 Kohlenstoffatomen,

$R^3$ je ein Wasserstoffatom oder je eine lineare oder verzweigte Alkylgruppe mit 1 bis 4 Kohlenstoffatomen und X einen Alkylenrest $-(CH_2)_n-$ mit n = 1, 2, 3 bedeuten,

dadurch gekennzeichnet, daß eine Verbindung der allgemeinen Formel II

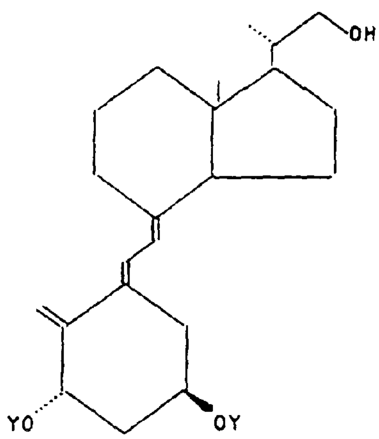

(II),

worin Y alkyl- oder arylsubstituierte Silylgruppen bedeuten, mit einer Verbindung der allgemeinen Formel III

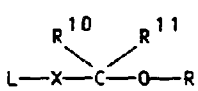

(III),

worin L für eine Abgangsgruppe Br, J, $CH_3$-$C_6H_4SO_2O$- ,

X für einen Alkylenrest $-(CH_2)_n-$ mit n = 1, 2 oder 3,

R für einen geradkettigen oder verzweigten Alkylrest mit 1 bis 8 Kohlenstoffatomen sowie

$R^{10}$ und $R^{11}$ ebenfalls je für einen Rest OR oder

$R^{10}$ und $R^{11}$ gemeinsam für ein Sauerstoffatom stehen,

unter Erhalt einer Verbindung der allgemeinen Formel IV

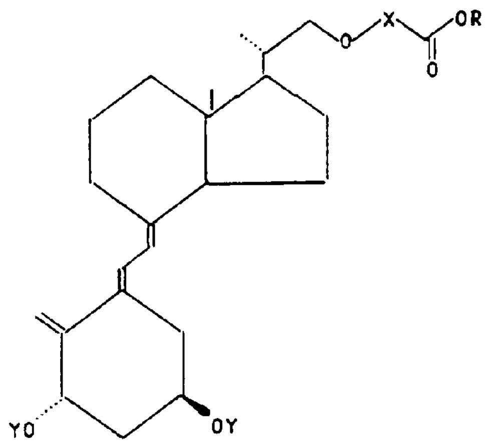

(IV),

verethert,
an deren Carbonylgruppe ein nucleophiles Reagenz der allgemeinen Formel V

$R^3$-Z (V),

worin $R^3$ eine lineare oder verzweigte Alkylgruppe mit 1 bis 4 Kohlenstoffatomen und Z MgHal (Hal = Cl, Br, J) oder ein Alkaliatom (Li, Na, K) bedeutet, addiert
oder wenn $R^3$ jeweils ein Wasserstoffatom sein soll, die Carbonylgruppe mit einem komplexen Hydrid, wie beispielsweise Lithiumaluminiumhydrid,
unter Bildung einer Verbindung der allgemeinen Formel VI

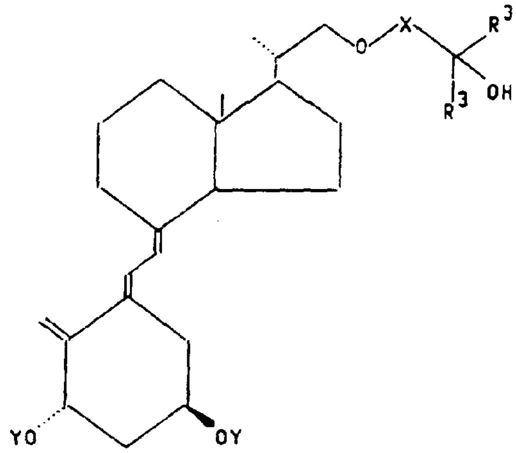

(VI),

umgesetzt und
diese durch photochemische Isomerisierung des Triensystems in Gegenwart eines Triplett-Sensibilisators in eine Verbindung der allgemeinen Formel VII

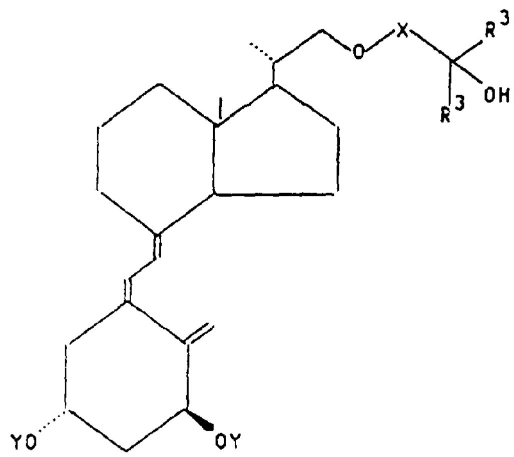

(VII),

umgewandelt wird sowie
die Silylschutzgruppen abgespalten und anschließend gegebenenfalls die freien Hydroxygruppen teilweise oder vollständig mit einem Carbonsäurechlorid oder -anhydrid, welche im Acylrest 1 bis 9 Kohlenstoffatome haben, verestert werden.

Die Addition des nucleophilen Reagenzes der allgemeinen Formel V an die Carbonylgruppe der Verbindung der allgemeinen Formel IV ist erfindungsgemäß unter Phasentransfer-Bedingungen durchzuführen.

Die Umwandlung einer Verbindung der allgemeinen Formel VI in eine Verbindung der allgemeinen Formel VII erfolgt z.B. durch Bestrahlung mit ultraviolettem Licht in Gegenwart eines sogenannten "Triplettensensibilisators". Im Rahmen der vorliegenden Erfindung wird hierfür Anthracen verwendet. Durch Spaltung der pi-Bindung der 5,6-Doppelbindung, Rotation des A-Ringes um 180° um die 5,6-Einfachbindung und Reetablierung der 5,6-Doppelbindung wird die Stereoisomerie an der 5,6-Doppelbindung umgekehrt.
Anschließend werden vorhandene Hydroxyschutzgruppen abgespalten, vorzugsweise unter Verwendung von Tetra-n-butyl-ammoniumfluorid sowie gewünschtenfalls die freien Hydroxygruppen nach gängigen Verfahren partiell oder vollständig mit dem entsprechenden Carbonsäurehalogenid (Halogenid = Chlorid, Bromid) oder Carbonsäureanhydrid verestert.

Die nachfolgenden Beispiele dienen der näheren Erläuterung der Erfindung.

Die zur Herstellung der 20(S)-Hydroxymethyl-Verbindung 2 benötigte 20(S)-Formylverbindung ist von M.J. Calverley in Tetrahedron 43, S. 4609, 1987, beschrieben.

**Beispiele:**

1α,3β-Bis[[dimethyl(1,1-dimethylethyl)silyl]oxy]-20(*S*)-hydroxymethyl-9,10-seco-5(*E*),7(*E*),10(19)-pregnatrien **2**

4,4g 1α,3β-Bis[[dimethyl(1,1-dimethylethyl)silyl]oxy]-20(*S*)-formyl-9,10-seco-5(*E*),7(*E*),10(19)-pregnatrien werden in 20 ml Ethanol und 2 ml Tetrahydrofuran gelöst und bei 0°C mit 100 mg Natriumborhydrid versetzt. Man rührt 1 Std. bei Raumtemperatur und gibt dann 20 ml Natriumchloridlösung zu. Es wird mit Methylenchlorid extrahiert, über Natriumsulfat getrocknet und eingeengt. Man erhält 4,4 g der Titelverbindung als farblose Kristalle.
$^1$H-NMR ($CDCl_3$): δ= 0,01 ppm (s, 12H, Si-$CH_3$); 0,51 (s, 3H, H-18); 0,81 u. 0,83 (s, je 9H, Si-t-butyl); 1,01 (d, J=7 Hz, 3H, H-21); 3,36 (m, 1H, H-22); 3,61 (ddbr, J=11, 5 Hz, 1H, H-22'); 4,17 (m, 1H, H-3); 4,48 (dd, J=10, 4 Hz, 1H, H-1); 4,89 u. 4,93 (s, je 1 H, H-19); 5,78 u. 6,40 (d, J=11 Hz, je 1H, H-6 u. H-7)
Schmelzpunkt: 107-109°C
$[\alpha]_D^{20}$: +54,2°; c=0,5; $CHCl_3$

1α,3β-Bis[[dimethyl(1,1-dimethylethyl]silyl]oxy]-23-oxa-9,10-seco-5(*E*),7(*E*),10(19)-cholatrien-24-carbonsäure-1,1-dimethylethylester **3**

2,5 g der Verbindung **2** werden in 50 ml Toluol gelöst, 6,3 g Bromessigsäure-tert.-butylester zugegeben und danach mit 20 ml 25% Natronlauge sowie 93 mg Tetrabutylammoniumhydrogensulfat versetzt. Man rührt nun 24 Std. bei Raumtemperatur, gibt erneut 50 mg des Katalysators zu und rührt weitere 6 Std. nach. Das Gemisch wird auf Natriumchloridlösung gegossen, mit Essigester extrahiert, über Natriumsulfat getrocknet und eingeengt. Das Produkt wird chromatographisch mit Hexan/Essigester an Kieselgel gereinigt, wobei man 1,375 g der Titelverbindung als farblosen Schaum erhält.

$^1$H-NMR ($CDCl_3$): δ= 0,01 ppm (s, 12H, Si-$CH_3$); 0,50 (s, 3H, H-18); 0,80 u. 0,84 (s, je 9H, Si-t-butyl); 1,03 (d, J=7 Hz, 3H, H-21); 1,42 (s, 9H. t-Butylester); 3,21 (dd, J=9, 7,5 Hz, 1H, H-22); 3,42 (m, 1H, H-22'); 3,88 (s, 2H, H-24); 4,18 (m, 1H, H-3); 4,49 m, 1H, H-1); 4,88 u. 4,94 (s, je 1H, H-19); 5,78 u. 6,40 (d, J=11 Hz, je 1H, H-6 u. H-7)

1α,3β-Bis[[dimethyl(1,1-dimethyl)silyl]oxy]-24-(1-ethyl-hydroxypropyl)-23-oxa-9,10-seco-5(*E*),7(*E*),10(19)-cholatrien **4a**

Aus 0,54 ml Bromethan und 174 mg Magnesiumspänen wird in 5 ml Tetrahydrofuran das Grignard-Reagenz bereitet. Bei 0°C gibt man nun 500 mg der Verbindung **3** in 2 ml Tetrahydrofuran zu und rührt 1 Std. nach. Es wird anschließend mit Ammoniumchloridlösung hydrolysiert, mit Essigester extrahiert, über Natriumsulfat getrocknet und eingeengt. Nach Chromatographie mit Hexan/Essigester an Kieselgel erhält man 246 mg der Titelverbindung als farblosen Schaum.
$^1$H-NMR ($CDCl_3$): δ= 0,01 ppm (s, 12H, Si-$CH_3$); 0,50 (s, 3H, H-18); 0,78 u. 0,82 (s, je 9H, Si-t-butyl); 0,81 u. 0,87 (t, J=7 Hz, je 3H, H-28 u. H-29); 0,98 (d, J=7 Hz, 3H, H-21); 3,13 (dd, J=9, 6 Hz, 1H, H-22); 3,14 (d, J=9,5 Hz, 1H, H-24); 3,22 (d, J=9,5 Hz, 1H, H-24'); 3,35 (dd, J=9, 3,5 Hz, 1H, H-22'); 4,15 (m, 1H, H-3); 4,48 (m, 1H, H-1); 4,88 u. 4,93 (s, je 1H, H-19); 5,77 u. 6,40 (d, J=11 Hz, je 1H, H-6 u. H-7)
$[\alpha]_D^{20}$: +62,3°, c=0,215, $CHCl_3$

1α;3β-Bis[[dimethyl(1,1-dimethylethyl)silyl]oxy]-24-(1-ethyl-1-hydroxy-propyl)-23-oxa-9,10-seco-5(*Z*),7(*E*),10(19)-cholatrien **5a**

246 mg **4a** werden zusammen mit 35 mg Anthracen und 10 µl Triethylamin in 80 ml Toluol gelöst und 6 Min. in einem Pyrex-Tauchreaktor mittels einer Quecksilberhochdrucklampe (Philips HPK 125) unter Stickstoffatmosphäre bestrahlt. Nach Einengen wird die Substanz an Kieselgel mit Hexan/Essigester chromatographiert, wobei man 202 mg der Titelverbindung als farblosen Schaum erhält.
$^1$H-NMR ($CDCl_3$): δ= 0,01 ppm (s, 12H, Si-$CH_3$); 0,50 (s, 3H, H-18); 0,83 (s, 18H, Si-t-butyl); 0,81 u. 0.85 (t, J=7 Hz, je 3H, H-28 u. H-29); 0,98 (d, J=7 Hz, 3H, H-21); 3,12 (dd, J=10, 6Hz, 1H, H-22); 3,13 (d, J=9,5 Hz, 1H, H-24); 3,22 (d, J=9,5 Hz, 1H, H-24'); 3,35 (dd, J=10, 3,5 Hz, 1H, H-22'); 4,13 (m, 1H, H-1); 4,31 (m, 1H, H-1); 4,80 u. 5,12 (s, je 1H, H-19); 5,96 u. 6,18 (d, J=11 Hz, je 1H, H-6 u. H-7)
$[\alpha]_D^{20}$: +41,3°, c=0,23, $CHCl_3$

24-(1-Ethyl-1-hydroxypropyl)-23-oxa-9,10-seco-5(*Z*),7(*E*),10(19)-cholatrien-1α,3β-diol **6a**

190 mg **5a** werden in 3,5 ml Tetrahydrofuran gelöst und mit 1,13 ml Tetrabutylammoniumfluoridlösung (1M in THF) versetzt. Man rührt 1 Std. bei 60°. Das Reaktionsgemisch wird nun 10 Min. mit Natriumhydrogencarbonatlösung gerührt, mit Essigester extrahiert, über Natriumsulfat getrocknet und eingeengt. Man reinigt das Produkt durch Chromatographie an Kieselgel mit Hexan/Essigester und erhält 66 mg der Titelverbindung als farblosen Schaum.
$^1$H-NMR ($CDCl_3$): δ= 0,56 ppm (s, 3H, H-18); 0,85 (t, J=7 Hz, 6H, H-28 u. H-29); 1,03 (d, J=7 Hz, 3H, H-21); 3,18 (dd, J=9, 5,5 Hz, 1H, H-22); 3,20 (d, J=10 Hz, 1H, H-24); 3,28 (d, J=10 Hz, 1H, H-24'); 3,40 (dd, J=9, 4,5 Hz, 1H, H-22'); 4,24 (m, 1H, H-3); 4,44 (m, 1H, H-1); 5,00 u. 5,32 (s, je 1H, H-19); 6,03 u. 6,38 (d, J=11Hz, je 1H, H-6 u. H-7)
$[\alpha]_D^{20}$: +20,3°, c=0,265, $CHCl_3$

1α-3β-Bis[[dimethyl(1,1-dimethylethyl)silyl]oxy]-24-(1-hydroxy-1-propylbutyl)-23-oxa-9,10-seco-5(*E*),7(*E*),10(19)-cholatrien **4b**

400 mg 3 werden dem aus 140 mg Magnesiumspänen und 0,52 ml 1-Brompropan bereiteten Grignard-Reagenz analog zu **4a** umgesetzt. Man erhält 160 mg der Titelverbindung als farblosen Schaum.
$^1$H-NMR ($CDCl_3$): δ= 0,01 ppm (s, 12H, Si-$CH_3$); 0,50 (s, 3H, H-18); 0,80 u. 0,85 (s, je 9H, Si-t-butyl); 0,81 u. 0,83 (t, J= 7Hz, 6H, H-30 u. H-31); 0,99 (d, J= 7 Hz, 3H, H-21); 3,08 (dd, J=9,7,5 Hz, 1H, H-22); 3,35 (m, 3H, H-22' u. H-24); 4,18 (m, 1H, H-3); 4,49 (m, 1H, H-1); 4,88 u. 4,94 (m, 1H, H-19); 5,78 u. 6,40 (d, J=11 Hz, je 1H , H-6 u. H-7)
$[\alpha]_D^{20}$: +55,2°, c=0,46, $CHCl_3$

1α,3β-Bis[[dimethyl(1,1-dimethylethyl)silyl]oxy]-24-(1-hydroxy-1-propylbutyl)-23-oxa-9,10-seco-5(*Z*),7(*E*),10(19)-cholatrien **5b**

150 mg **4b** werden mit 20 mg Anthracen und 5,8 µl Triethylamin analog zu **5a** umgesetzt, wobei man 125 mg der Titelverbindung als farblosen Schaum erhält.
$^1$H-NMR ($CDCl_3$): δ= 0,01 ppm (s, 6H, Si-$CH_3$); 0,49 (s, 3H, H-18); 0,80 (s, 18H, Si-t-butyl); 0,80 (t, J=7 Hz, 6H, H-30

u. H-31); 0,96 (d, J=7 Hz, 3H, H-21); 3,06 (dd, J= 8,5, 7,5 Hz, 1H, H-22); 3,34 (m, 3H, H-22' u- H-24); 4,13 (m, 1H, H-3); 4,30 (m, 1H, H-1): 4,80 u. 5,12 (s, je 1H, H-19); 5,96 u. 6,18 (d, J=11Hz, je 1H, H-6 u. H-7)
$[\alpha]_D^{20}$: +36,8°, c=0,22, $CHCl_3$

24-(1-Hydroxy-1-propylbutyl)-23-oxa-9,10-seco-5(*Z*),7(*E*),10(19)-cholatrien-1α,3β-diol **6b**

120 mg **5b** werden mit 1,2 ml Tetrabutylammoniumfluoridlösung (1M in THF) analog zu 6a umgesetzt. Man erhält 47 mg der Titelverbindung als farblosen Schaum.
$^1$H-NMR ($CDCl_3$): δ= 0,57 ppm (s, 3H, H-18); 0,91 (t, J=7 Hz, 6H, H-30 u. H-31); 1,04 (d, J=7 Hz 3H, H-21); 3,14 (dd, J=10, 6 Hz, 1H, H-22); 3,18 (d, J=9 Hz, 1H, H-24); 3,26 (d, J=9 Hz, 1H, H-24'); 3,40 (dd, J=10, 4 Hz, 1H, H-22); 4,24 (m, 1H, H-3); 4,44 (m, 1H, H-1); 5,00 u. 5,33 (s, je 1H, H-19); 6,03 u. 6,38 (d, J=11 Hz, je 1H, H-6 u. H-7)
$[\alpha]_D^{20}$: +22,2°, c=0,175, $CHCl_3$

1α,3β-Bis[[dimethyl(1,1-dimethylethyl)silyl]oxy]-24-(1-hydroxy-1-methylethyl)-23-oxa-9,10-seco-5(*E*),7(*E*),10(19)-cholatrien **4c**

820 mg 3 werden mit dem Grignard-Reagenz aus 290 mg Magnesiumspänen und 0,74 ml Methyliodid in 13 ml Diethylether analog zu **4a** umgesetzt. Man erhält 360 mg der Titelverbindung als farblosen Schaum.
$^1$H-NMR ($CDCl_3$): δ= 0,01 ppm (s, 12H, Si-$CH_3$); 050 (s, 3H, H-18); 0,81 u. 0,83 (s, je 9H, Si-t-butyl); 1,00 (d, J=7 Hz, 3H, H-21); 1,16 (s, 6H, H-26 u. H-27); 3,11 (d, J=9,5 Hz, 1H, H-24); 3,16 (m, 1H, H-22); 3,21 (d, J=9,5 Hz, 1H, H-24'); 3,48 (dd, J=9, 4 Hz, 1H, H-22'); 4,18 (m, 1H, H-3); 4,48 (m, 1H, H-1); 4,89 u. 4,94 (s, je 1H, H-19); 5,78 u. 6,40 (d, J=11Hz, je 1H, H-6 u. H-7)
$[\alpha]_D^{20}$: +51,2°, c=0,505, $CHCl_3$

1α,3β-Bis[[dimethyl(1,1-dimethylethyl)silyl]oxy]-24-(1-hydroxy-1-methylethyl)-23-oxa-9,10-seco-5(*Z*),7(*E*),10(19)-cholatrien **5c**

330 mg **4c** werden mit 50 mg Anthracen und 15 µl Triethylamin in 80 ml Toluol analog zu **5a** umgesetzt, wobei man 280 mg der Titelverbindung als farblosen Schaum erhält. $^1$H-NMR ($CDCl_3$): δ= 0,01 ppm (s, 12H, Si-$CH_3$); 0,49 (s, 3H, H-18); 0,82 (s, 18H, Si-t-butyl); 0,99 (d, J= 7Hz, 3H, H-21); 1,15 (s, 6H, H-26 u. H-27); 3,11 (d, J= 9,5Hz, 1H, H-24); 3,17 (m, 1H, H-22); 3,20 (d, J=9,5 Hz, 1H, H-24'); 3,38 (dd, J=9, 4 Hz, 1H, H-22'); 4,14 (m, 1H, H-3); 4,31 (m, 1H, H-1); 4,81 u. 5,12 (s, je 1H, H-19); 5,96 u. 6,19 (d, J= 11Hz, je 1H, H-6 u. H-7)
$[\alpha]_D^{20}$: +46,6°, c=0,12, $CHCl_3$

24-(1-Hydroxy-1-methylethyl)-23-oxa-9,10-seco-5(*Z*),7(*E*),10(19)-cholatrien-1α,3β-diol **6c**

260 mg **5c** werden mit 1,6 ml Tetrabutylammoniumfluoridlösung (1M in THF) analog zu **6a** umgesetzt. Es werden 117 mg der Titelverbindung als farbloser Schaum erhalten.
$^1$H-NMR ($CDCl_3$): δ= 0,56 ppm (s, 3H, H-18); 1,04 (d, J=7 Hz, 3H, H-21); 1,22 (s, 6H, H-26 u. H-27); 3,16 (d , J=9 Hz, 1H, H-24); 3,22 (m, 1H, H-22); 3,26 (d, J=9 Hz, 1H, H-24'); 3,43 (dd, J=9, 4,5 Hz, 1H, H-22'); 4,22 (m, 1H, H-3); 4,43 (m, 1H, H-1); 5,01 u. 5,33 (s, je 1H, H-19), 6,03 u. 6,39 (d, J=11 Hz, je 1H, H-6 u. H-7)
$[\alpha]_D^{20}$: +22°, c=0,15, $CHCl_3$

3-[1α,3β-Bis[[dimethyl(1,1-dimethylethyl)silyl]oxy]-23-oxa-9,10-seco-5(*E*),7(*E*);10(19)-cholatrien-24-yl]-propansäuremethylester **7**

2,3 g 2 werden mit 3,75 g Brombuttersäure-Orthotrimethylester und 212 mg Tetrabutylammomiumhydrogensulfat in 5,4 ml Natronlauge (50%) über Nacht bei Raumtemperatur gerührt. Man verdünnt mit Wasser, extrahiert mit Essigester, wäscht mit Natriumchloridlösung, trocknet über Natriumsulfat und engt ein. Der Rückstand wird an Kieselgel mit Hexan/Essigester chromatographiert, wobei 1,42 g der Titelverbindung anfallen.
$^1$H-NMR ($CDCl_3$): δ= 0,01 ppm (s, 12H, Si-$CH_3$); 0,50 (s, 3H, H-18); 0,80 u. 0,82 (s, je 9H, Si-t-butyl); 0,96 (d, J=7 Hz, 3H, H-21); 2,11 (q, J= 7 Hz, 2H, H-25); 2,36 (t, J=7 Hz, 2H, H-26); 3,32 (m, 2H, H-22); 3,40 (t, J=7 Hz, 2H, H-24); 3,61 (s, 3H, Methylester); 4,17 (m, 1H, H-3); 4,48 (m, 1H, H-1); 4,88 u. 4,93 (s, je 1H, H-19); 5,76 u. 6,40 (d, J=11 Hz je 1H, H-6 u. H-7)
$[\alpha]_D^{20}$: +52,7°, c=0,4, $CHCl_3$

1α,3β-Bis[[dimethyl(1,1-dimethylethyl)silyl]oxy]-24-(3-hydroxy-3-methylbutyl)-23-oxa-9,10-seco-5(*E*),7(*E*),10(19)-cholatrien **8a**

Das Grignard-Reagenz aus 253 mg Magnesiumspänen und 0,65 ml Methyliodid in 13 ml Diethylether wird mit 700 mg **7** analog zu **4a** umgesetzt, man erhält 380 mg der Titelverbindung als farblosen Schaum.
$^1$H-NMR ($CDCl_3$): δ= 0,08 ppm (s, 12H, Si-$CH_3$); 0,55 (s, 3H, C-18); 0,89 (s, 18H, Si-t-butyl); 1,04 (d, J=7 Hz, 3H, H-21); 1,22 (s, 6H, H-28 u. H-29); 3,13 u. 3,43 (m, 4H, H-22 u. H-24); 4,22 (m, 1H, H-3); 4,54 (m, 1H, H-1); 4,96 u. 5.00 (s, je 1H, H-19); 5,83 u. 6,46 (d, je 1H, H-6 u. H-7)
$[\alpha]_D^{20}$: +28,1°, c=0,515, $CHCl_3$

1α,3β-Bis[[dimethyl(1,1-dimethylethyl)silyl]oxy]-24-(3-hydroxy-3-methylbutyl)-23-oxa-9,10-seco-5(*Z*),7(*E*),10(19)-cholatrien **9a**

370 mg **8a** werden mit 88 mg Anthracen und 20 µl Triethylamin in 80 ml Toluol analog zu **5a** umgesetzt. Man erhält 221 mg der Titelverbindung als farblosen Schaum.
$^1$H-NMR ($CDCl_3$): δ= 0,01 ppm (s, 12H, Si-$CH_3$); 0,48 (s, 3H, H-18); 0,80 (s, 18H, Si-t-butyl); 0,98 (d, J=7 Hz, 3H, H-21); 1,16 (s, 6H, H-28 u. H-29); 3,06 (dd, J=8,5, 7,5 Hz, 1H, H-22); 3,35 (m, 3H, H-22' u. H-24); 4,13 (m, 1H, H-1); 4,31 (m, 1H, H-1); 4,80 u. 5,11 (s, je 1H, H-19); 5,96 u. 6,17 (d, J=11 Hz, je 1H, H-6 u. H-7)
$[\alpha]_D^{20}$: +24°, c=0,345, $CHCl_3$

24-(3-Hydroxy-3-methylbutyl)-23-oxa-9,10-seco-5(*Z*),7(*E*),10(19)-cholatrien-1α,3β-diol **10a**

200 mg **9a** werden mit 1,13 ml Tetrabutylammoniumfluoridlösung (1M in THF) analog **6a** umgesetzt. Man erhält 113 mg der Titelverbindung.
$^1$H-NMR ($CDCl_3$): δ= 0,55 (s, 3H, H-18); 1,03 (d, J=7 Hz, H-21); 1,20 (s, 6H, H-28 u. H-29); 3,12 (dd, J=8.5, 73 Hz, 1H, H-22); 3,39 (m, 3H, H-22' u. H-24); 4,22 (m, 1H, H-3); 4,43 (m, 1H, H-1); 5,00 u. 5,32 (s, je 1H, H-19); 6,02 u. 6,38 (d, J=11 Hz, je 1H, H-6 u. H-7)
$[\alpha]_D^{20}$: +20,9°, c=0,415, $CHCl_3$

1α,3β-Bis[[dimethyl(1,1-dimethylethyl)silyl]oxy]-24-(3-ethyl-3-hydroxypentyl)-23-oxa-9,10-seco-5(*E*),7(*E*),10(19)-cholatrien **8b**

700 mg 7 werden mit dem Grignard-Reagenz aus 253 mg Magnesiumspänen und 0,78 ml Bromethan in 13 ml THF analog **4a** umgesetzt. Es werden 360 mg der Titelverbindung als farbloser Schaum erhalten.
$^1$H-NMR ($CDCl_3$): δ= 0,01 ppm (s, 12H, Si-$CH_3$); 0,50 (s, H, H-18); 0,80 u. 0,85 (s, je 9H, Si-t-butyl); 0,81 u. 0,83 (t, J=7 Hz, je 3H, H-30 u. H-31); 0,99 (d, J=7 Hz, H-21); 3,08 (dd, J=9, 7,5 Hz, 1H, H-22); 3,35 (m, 3H, H-22' u. H-24); 4,18 (m, 1H, H-3); 4,49 (m, 1H, H-1); 4,88 u. 4,94 (s, je 1H, H.19); 5,78 u. 6,40 (d, J=11 Hz, je 1H, H-6 u- H-7)
$[\alpha]_D^{20}$: +55,2°, c=0,46, $CHCl_3$

1α,3β-Bis[[dimethyl(1,1-dimethylethyl)silyl]oxy]-24-(3-ethyl-3-hydroxypentyl)-23-oxa-9,10-seco-5(*Z*),7(*E*),10(19)-cholatrien **9b**

350 mg **8b** werden mit 80 mg Anthracen und 18 µl Triethylamin analog **5b** umgesetzt. Man erhält 300 mg der Titelverbindung als farblosen Schaum.
$^1$H-NMR ($CDCl_3$): δ= 0,01 ppm (s, 12H, Si-$CH_3$); 0,49 (s, 3H, H-18); 0,80 (s, 18H, Si-t-butyl); 0,80 (t, J=7 Hz, 6H, H-30 u. H-31); 0,96 (d, J=7 Hz, 3H, H-21); 3,06 (dd, J=8,5, 7,5 Hz, 1H, H-22); 3,34 (m, 3H, H-22' u- H-24); 4,13 (m, 1H, H-3); 4,30 (m, 1H, H-1); 4,80 u. 5,12 (s, je 1H, H-19); 5,96 u. 6,18 (d J=11 Hz, je 1H, H-6 u. H-7)
$[\alpha]_D^{20}$: +38,6°, c=0,22, $CHCl_3$

24-(3-Ethyl-3-hydroxypentyl)-23-oxa-9,10-seco-5(*Z*),7(*E*),10(19)-cholatrien-1α,3β-diol **10b**

290 mg **9b** werden mit 1,54 ml Tetrabutylammoniumfluoridlösung (1M in THF) analog zu **6b** umgesetzt. Es werden 106 mg der Titelverbindung als farbloser Schaum erhalten.
$^1$H-NMR ($CDCl_3$): δ= 0,55 (s, 3H, H-18); 0,84 (t, J=7 Hz, 6H, H-30 u. H-31); 1,07 (d, J=7 Hz, 3H, H-21); 1,48 (q, J=7 Hz, 4H, H-28 u. H-29); 3,12 (dd, J=8,5, 7,5 Hz, 1H, H-22); 3,40 (m, 3H, H-22' u. H-24); 4,22 (m, 1H, H-3); 4,43 (m, 1H, H-1); 5,00 u. 5,33 (s, je 1H, H-19); 6,03 u. 6,38 (d, J=11 Hz, je 1H, H-6 u. H-7)
$[\alpha]_D^{20}$: +18,4°, c=0,13, $CHCl_3$

1α-3β-Bis[[dimethyl(1,1-dimethylethyl)silyl]oxy]-24-(1-ethyl-hydroxy-propyl)-23-oxa-9,10-seco-5(*E*),7(*E*),10(19)-cholatrien **4a**

Versuch der Darstellung analog Hesse US-Patent 4,772,433 bzw. EP 0078704:

410 mg 2 werden mit 1,4 g 1,2-Epoxy-2-ethylbutan [Darstellung analog J.S. Ng Synthetic Communications 20, 1193 (1990)], 50 mg Dibenzo-18-crown-6 und 252 mg Kalium-tert-Butanolat in 5 ml Benzol 55 min zum Rückfluß erhitzt. Es wird mit Wasser vedünnt, mit Methylenchlorid extrahiert, mit Natriumhydrogencarbonatlösung und Natriumchloridlösung gewaschen und über Natriumsulfat getrocknet. Nach Abziehen des Lösungsmittels wird der Rückstand an Kieselgel mit Hexan/Essigester gereinigt, wobei als einziges charakterisierbares Produkt 30 mg des Ausgangsmaterials anfallen.

Tabelle 1

| | Stimulierung des Stratum granulosum | $Ca^{2+}$-Serum |
|---|---|---|
| Verbindung **6b** (Erfindung) | ..............41 %................. | 2,75 mmol/l |
| Verbindung **6c** (Vergleich) | ..............41 %................. | 3,645 mmol/l |
| Lösungsmittelkontrolle (Ethanol/Isopropylmyristat 95 / 5 V/V) | ...............-................. | 2,73 mmol/l |

## Patentansprüche

**1.** 23-Oxa-Derivate in der Vitamin D-Reihe der Formel I

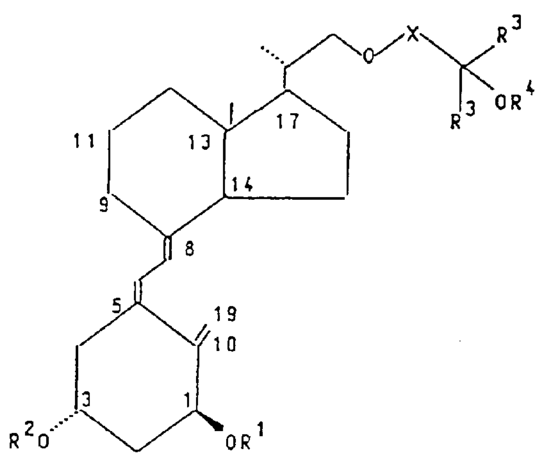

(I),

worin

$R^1$, $R^2$ und $R^4$ unabhängig voneinander ein Wasserstoffatom oder eine Acylgruppe mit 1 bis 9 Kohlenstoffatomen,

$R^3$ je ein Wasserstoffatom oder je eine lineare oder verzweigte Alkylgruppe mit 1 bis 4 Kohlenstoffatomen und X einen Alkylenrest-$(CH_2)_n$- mit n = 1, 2, 3, wobei wenn n = 1 $R^3$ nicht je eine Methyl- oder Propylgruppe, letztere in Verbindung mit $R^1$, $R^2$ und $R^4$ in der Bedeutung je einer $C_1$-$C_9$-Acylgruppe sein kann, bedeuten,

**2.** 24-(1-Ethyl-1-hydroxypropyl)-23-oxa-9,10-seco-5(Z),7(E),10(19)-cholatrien1α,3β-diol,
24-(1-Hydroxy-1-propylbutyl)-23-oxa-9,10-seco-5(Z),7(E),10(19)-cholatrien-1α,3β-diol,
24-(3-Hydroxy-3-methylbutyl)-23-oxa-9,10-seco-5(Z),7(E),10(19)-cholatrien-1α,3β-diol,
24-(3-Ethyl-3-hydroxypentyl)-23-oxa-9,10-seco-5(Z),7(E),10(19)-cholatrien-1α,3β-diol.

**3.** Verfahren zur Herstellung von Verbindungen der allgemeinen Formel I'

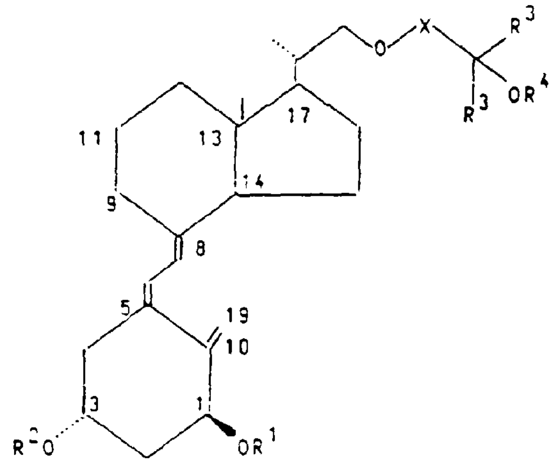

(I´)

worin

$R^1$, $R^2$ und $R^4$ unabhängig voneinander ein Wasserstoffatom oder eine Acylgruppe mit 1 bis 9 Kohlenstoffatomen,

$R^3$ je ein Wasserstoffatom oder je eine lineare oder verzweigte Alkylgruppe mit 1 bis 4 Kohlenstoffatomen und X einen Alkylenrest $-(CH_2)_n-$ mit n = 1, 2, 3 bedeuten,

dadurch gekennzeichnet, daß eine Verbindung der allgemeinen Formel II

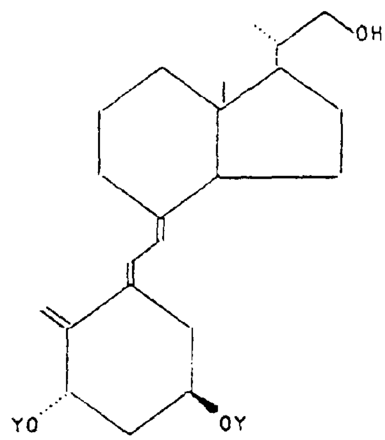

(II),

worin Y alkyl- oder arylsubstituierte Silylgruppen bedeuten, mit einer Verbindung der allgemeinen Formel III

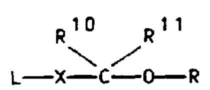

(III),

worin L für eine Abgangsgruppe Br, J, $CH_3-C_6H_4SO_2O-$,

X für einen Alkylenrest $-(CH_2)_n-$ mit n = 1, 2 oder 3,

R für einen geradkettigen oder verzweigten Alkylrest mit 1 bis 8 Kohlenstoffatomen sowie

$R^{10}$ und $R^{11}$ ebenfalls je für einen Rest OR oder

$R^{10}$ und $R^{11}$ gemeinsam für ein Sauerstoffatom stehen, unter Erhalt einer Verbindung der allgemeinen Formel IV

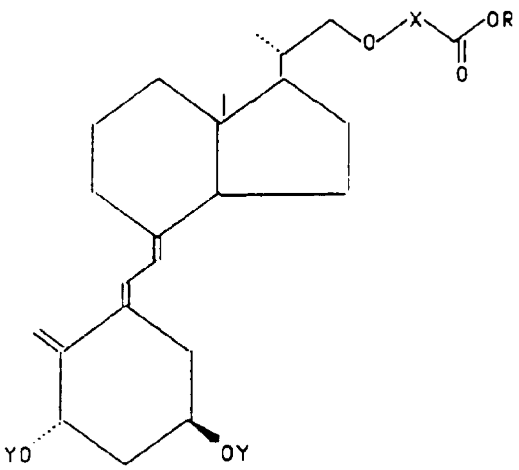

(IV),

verethert,
an deren Carbonylgruppe ein nucleophiles Reagenz der allgemeinen Formel V

$R^3$-Z (V),

worin $R^3$ eine lineare oder verzweigte Alkylgruppe mit 1 bis 4 Kohlenstoffatomen und Z MgHal (Hal = Cl, Br, J) oder ein Alkaliatom (Li, Na, K) bedeutet, addiert
oder wenn $R^3$ jeweils ein Wasserstoffatom sein soll, die Carbonylgruppe mit einem komplexen Hydrid, wie beispielsweise Lithiumaluminiumhydrid, unter Bildung einer Verbindung der allgemeinen Formel VI

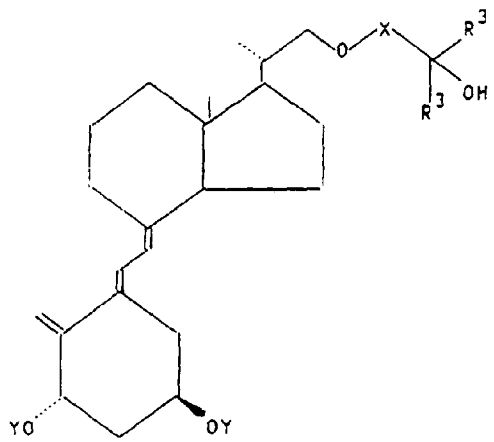

(VI),

umgesetzt und
diese durch photochemische Isomerisierung des Triensystems in Gegenwart eines Triplett-Sensibilisators in eine Verbindung der allgemeinen Formel VII

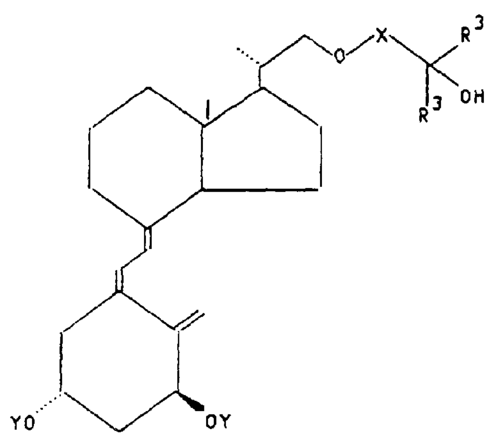

(VII),

umgewandelt wird sowie
die Silylschutzgruppen abgespalten und anschließend gegebenenfalls die freien Hydroxygruppen teilweise oder

vollständig mit einem Carbonsäurechlorid oder -anhydrid, welche im Acylrest 1 bis 9 Kohlenstoffatome haben, verestert werden.

**4.** Pharmazeutische Präparate, die mindestens eine Verbindung der allgemeinen Formel I sowie einen pharmazeutisch verträglichen Träger enthalten.

**5.** Verwendung der Verbindungen der allgemeinen Formel I zur Herstellung von Arzneimitteln.

## Claims

**1.** 23-Oxa derivatives in the vitamin D series of formula I

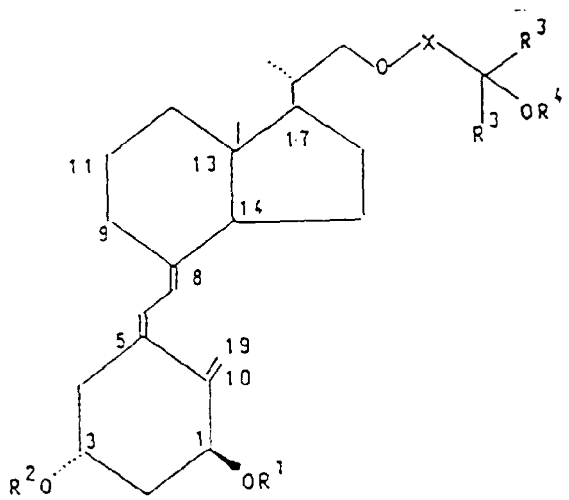

(I),

wherein

| | |
|---|---|
| $R^1$, $R^2$ and $R^4$, | each independently of the others, represent a hydrogen atom or an acyl group having from 1 to 9 carbon atoms, |
| each $R^3$ | represents a hydrogen atom or a linear or branched alkyl group having from 1 to 4 carbon atoms, and X represents an alkylene radical $-(CH_2)_n-$ wherein n is 1, 2 or 3 with the proviso that if n is 1, each $R^3$ may not be a methyl or propyl group, the latter in conjunction with $R^1$, $R^2$ and $R^4$ each representing a $C_1$-$C_9$-acyl group. |

**2.** 24-(1-Ethyl-1-hydroxypropyl)-23-oxa-9,10-seco-5(Z),7(E),10(19)-cholatriene-1α,3β-diol, 24-(1-hydroxy-1-propylbutyl)-23-oxa-9,10-seco-5(Z),7(E),10(19)-cholatriene-1α,3β-diol, 24-(3-hydroxy-3-methylbutyl)-23-oxa-9,10-seco-5(Z),7(E),10(19)-cholatriene-1α,3β-diol, and 24-(3-ethyl-3-hydroxypentyl)-23-oxa-9,10-seco-5(Z),7(E),10(19)-cholatriene-1α,3β-diol.

**3.** Process for the preparation of compounds of the general formula I'

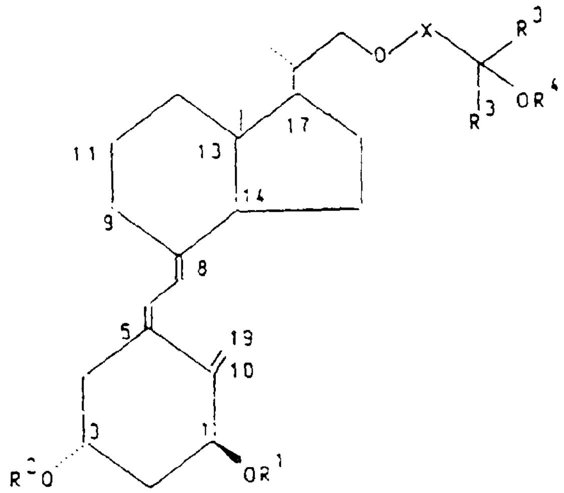

(I'),

wherein

R¹, R² and R⁴, each independently of the others, represent a hydrogen atom or an acyl group having from 1 to 9 carbon atoms,

each $R^3$ represents a hydrogen atom or a linear or branched alkyl group having from 1 to 4 carbon atoms and X represents an alkylene radical $-(CH_2)_n-$ wherein n = 1, 2 or 3, characterised in that a compound of the general formula II

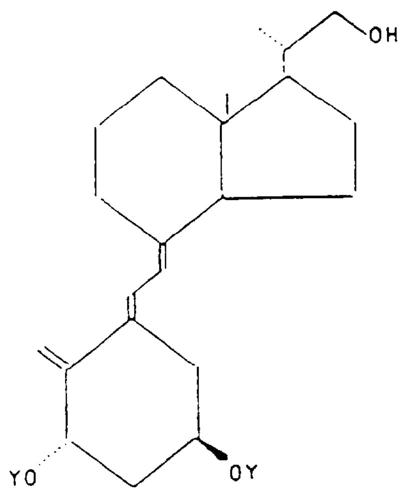

(II),

wherein Y represents alkyl- or aryl-substituted silyl groups, is etherified with a compound of the general formula III

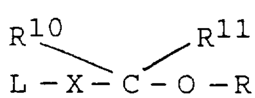

(III),

wherein L represents a leaving group Br, I or $CH_3$-$C_6H_4SO_2O$-,

X represents an alkylene radical $-(CH_2)_n-$ wherein n = 1, 2 or 3,

R represents a straight-chained or branched alkyl radical having from 1 to 8 carbon atoms, and

$R^{10}$ and $R^{11}$ likewise each represent a radical OR or

$R^{10}$ and $R^{11}$ together represent an oxygen atom, yielding a compound of the general formula IV

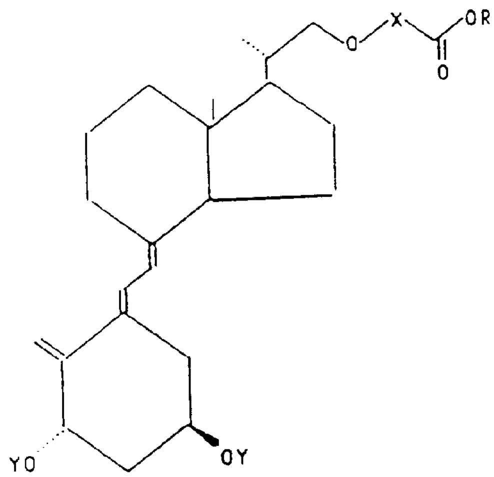

(IV),

to the carbonyl group of which is added a nucleophilic reagent of the general formula V

$R^3$-Z (V),

wherein $R^3$ represents a linear or branched alkyl group having from 1 to 4 carbon atoms and Z represents MgHal (Hal = Cl, Br, I) or an alkali metal atom (Li, Na, K), or if each $R^3$ is to be a hydrogen atom, the carbonyl group is reacted with a complex hydride, such as, for example, lithium aluminium hydride, to form a compound of IIthe general formula VI

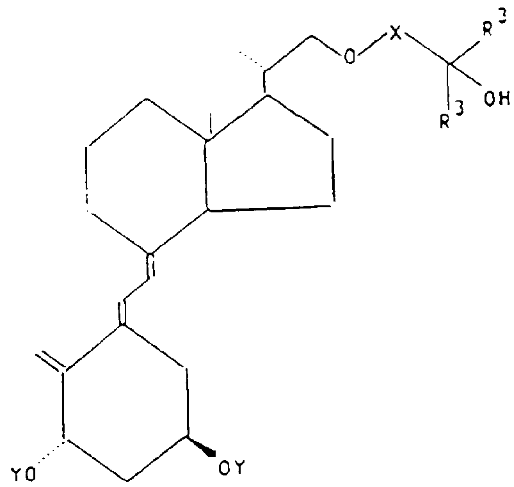

(VI),

and that compound is converted by photochemical isomerisation of the triene system in the presence of a triplet sensitiser into a compound of the general formula VII

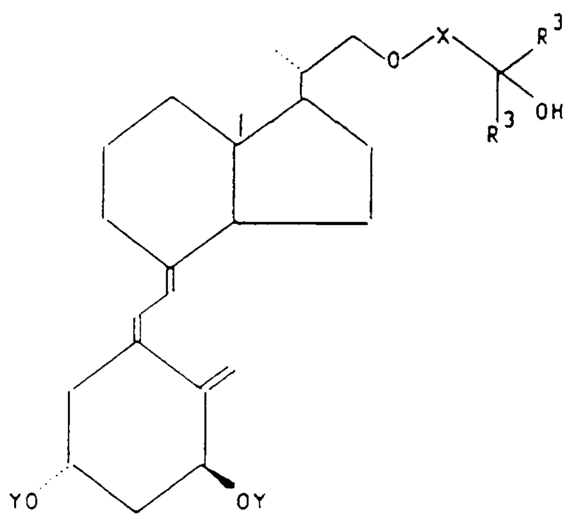

(VII),

and

the silyl protecting groups are removed and then, optionally, the free hydroxy groups are partially or completely esterified with a carboxylic acid chloride or anhydride that has from 1 to 9 carbon atoms in the acyl radical.

**4.** Pharmaceutical preparations that comprise at least one compound of the general formula I and a pharmaceutically acceptable carrier.

**5.** Use of the compounds of the general formula I for the preparation of medicaments.

**Revendications**

**1.** Dérivés de 23-oxa de la série de la vitamine-D de formule I

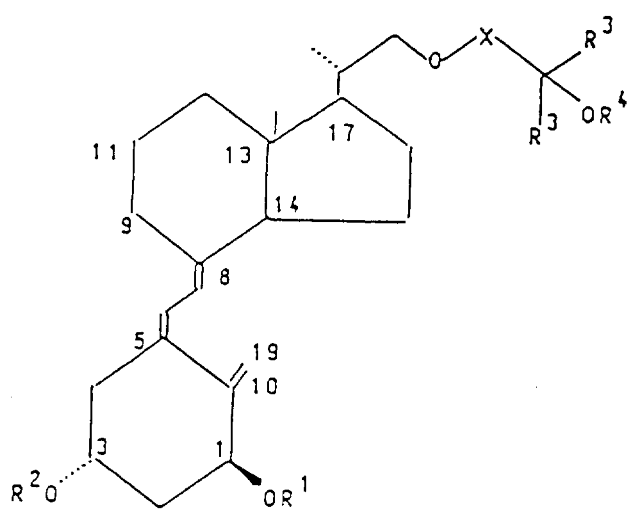

(I),

dans laquelle

| | |
|---|---|
| $R^1$, $R^2$ et $R^4$, | indépendamment les uns des autres représentent un atome d'hydrogène ou un groupe acyle avec 1 à 9 atomes de carbone, |
| $R^3$ | chacun représente un atome d'hydrogène ou un groupe alkyle linéaire ou ramifié avec 1 à 4 atomes de carbone et X représente un radical alkylène $-(CH_2)_n-$ avec n valant 1, 2, 3 lorsque n étant égal à 1, $R^3$ ne pouvant pas être un groupe méthyle ou propyle, ce dernier composé relatif à $R^1$, $R^2$ et $R^4$, ayant la signification d'un groupe acyle en $C_1$-$C_9$, |

**2.** 24-(1-éthyl-1-hydroxypropyl)-23-oxa-9,10-séco-5(Z),7(E), 10(19)-cholatriène-1α,3β-diol,

24-(1-hydroxy-1-propylbutyl)-23-oxa-9,10-séco-5(Z),7(E), 10(19)-cholatriène-1α,3β-diol,
24-(3-hydroxy-3-méthylbutyl)-23-oxa-9,10-séco-5(z), 7(E), 10(19)-cholatriène-1α,3β-diol,
24-(3-éthyl-1-hydroxypentyl)-23-oxa-9,10-séco-5(Z), 7(E), 10(19)-cholatriène-1α,3β-diol.

**3.** Procédé pour la préparation de composés de formule générale I'

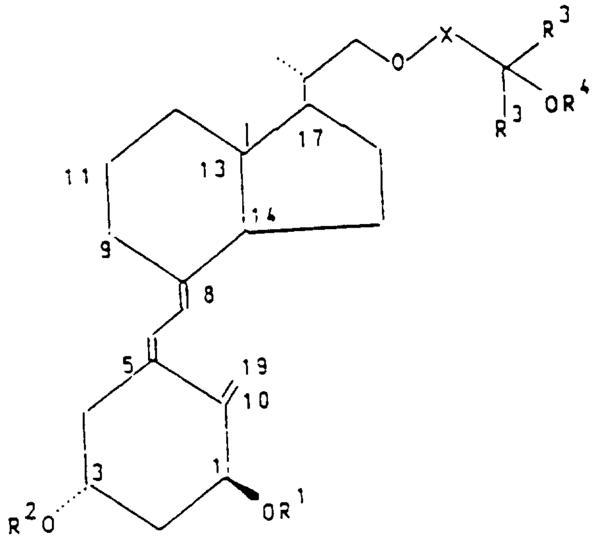

(I')

dans laquelle

$R^1$, $R^2$ et $R^4$, indépendamment les uns des autres, représentent un atome d'hydrogène ou un groupe acyle avec 1 à 9 atomes de carbone,

$R^3$ représente chacun un atome d'hydrogène ou un groupe alkyle linéaire ou ramifié avec 1 à 4 atomes de carbone et X représente un radical alkylène $-(CH_2)_n-$ avec n = 1, 2, 3,

caractérisé en ce qu'on éthérifie un composé de formule générale II

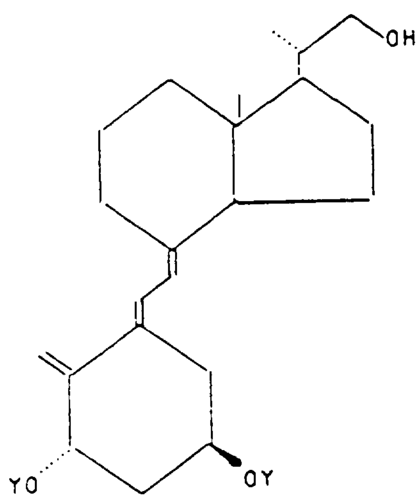

(II),

dans laquelle
Y représente un groupe silyle, alkyle ou aryle substitué, avec un composé de formule générale III

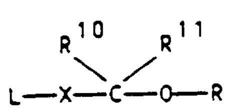

(III),

dans laquelle

| | |
|---|---|
| L | représente un groupe éliminable Br, J, $CH_3$-$C_6H_4SO_2O$-, |
| X | représente un radical alkylène $-(CH_2)_n-$ avec n = 1, 2 ou 3, |
| R | représente un radical alkyle linéaire ou ramifié avec 1 à 8 atomes de carbone ainsi que |
| $R^{10}$ et $R^{11}$ | également chacun représente un radical OR ou |
| $R^{10}$ et $R^{11}$ | ensemble représentent un atome d'oxygène, |

en obtenant un composé de formule générale IV

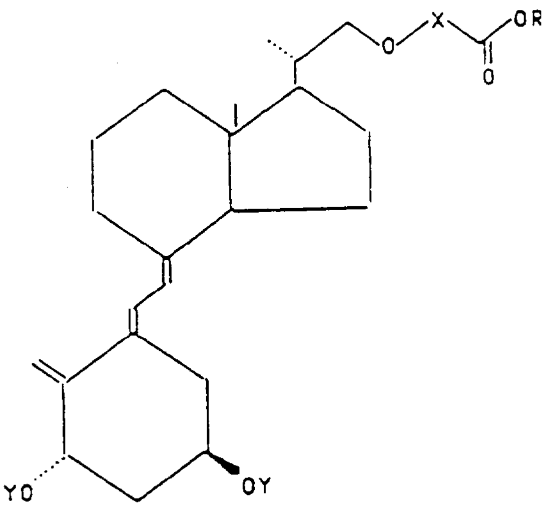

(IV),

au groupe carbonyle duquel on additionne un réactif nucléophile de formule générale V

$R^3$-Z (V)

dans laquelle

$R^3$ représente un groupe alkyle linéaire ou ramifié avec 1 à 4 atomes de carbone et Z représente MgHal (Hal = Cl, Br, J) ou un atome de métal alcalin (Li, Na, K) ou lorsque $R^3$ doit chacun représenter un atome d'hydrogène, on fait réagir le groupe carbonyle avec un hydrure complexe comme par exemple l'hydrure de lithium-aluminium en formant un composé de formule générale VI

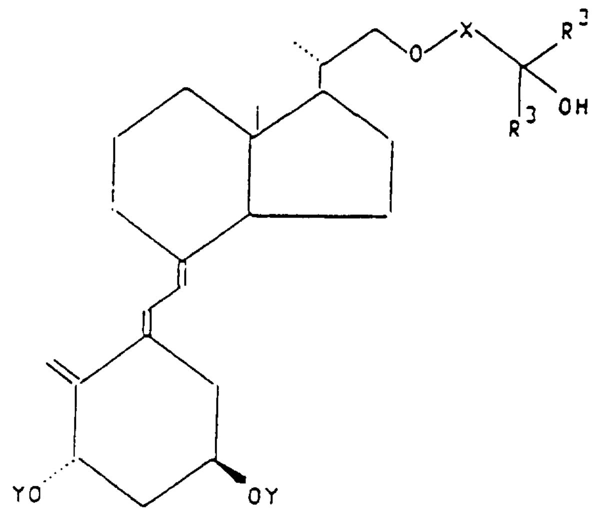

(VI),

et on transforme celui-ci par isomérisation photochimique du système des triènes en présence d'un sensibilisateur du triplet en un composé de formule générale VII

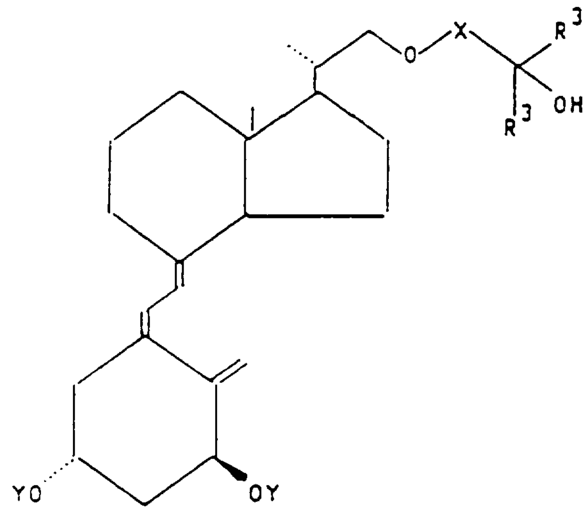

(VII),

ainsi que
on sépare les groupes protecteurs silyles et ensuite éventuellement on estérifie les groupes hydroxyles libres partiellement ou entièrement avec un chlorure d'acide carboxylique ou anhydride d'acide carboxylique qui contiennent dans le radical acyle 1 à 9 atomes de carbone.

**4.** Préparation pharmaceutique qui contient au moins un composé de formule générale I ainsi qu'un véhicule pharmaceutiquement compatible.

**5.** Utilisation des composés de formule générale I pour la préparation de médicaments.